# EUROPÄISCHE PATENTSCHRIFT

(11) **EP 0 635 505 B1**
(45) Veröffentlichungstag und Bekanntmachung des Hinweises auf die Patenterteilung: **15.10.1997**
(21) Anmeldenummer: 94110781.5
(22) Anmeldetag: 12.07.1994
(51) Int. Cl.: C07D 413/06, C07D 413/14, A61K 31/42, A61K 31/445

(54) **4-Aryloxy- und 4-Arylthiopiperidin-derivate**
4-Aryloxy- and 4-arylthiopiperidine derivatives
Dérivés de 4-aryloxy et 4-arylthio-piperidine

(30) Priorität: 21.07.1993 DE 4324393
(43) Veröffentlichungstag der Anmeldung: 25.01.1995
(73) Patentinhaber: MERCK PATENT GmbH, 64293 Darmstadt (DE)
(72) Erfinder: Prücher, Helmut, D-64646 Heppenheim (DE); Gottschlich, Dr. Rudolf, D-64354 Reinheim (DE); Bartoszyk, Gerd, D-64289 Darmstadt (DE); Seyfried, Dr. Christoph, D-64342 Jugenheim (DE)

(56) Entgegenhaltungen:
- EP-A- 0 300 272
- EP-A- 0 443 197
- EP-A- 0 459 256
- BIOORGANIC AND MEDICINAL CHEMISTRY LETTERS Bd. 2, Nr. 2 , 1992 , UK Seiten 165 - 170 H.PRÜCHER ET AL '(5S)-3-Aryl-5(1-piperidin ylmethyl)-2-oxazolidinones, a new class of potential neuroleptics with a high affinity for sigma receptors.'
- JOURNAL OF MEDICINAL CHEMISTRY Bd. 13, Nr. 1 , 1970 Seiten 1 - 6 R.L.DUNCAN ET AL 'Aroylpiperidines and pyrrolidines'

## Beschreibung

Die Erfindung betrifft 4-Aryloxy- oder 4-Arylthiopiperidinderivate der Formel I 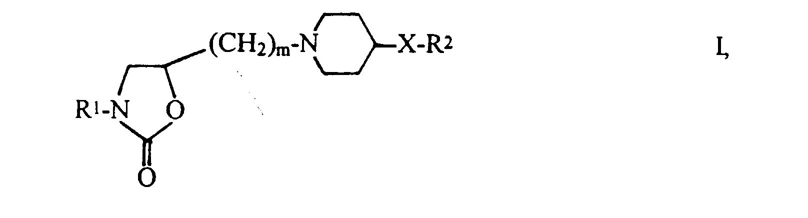 worin
- R¹ und R²: jeweils unabhängig voneinander unsubstituierte oder ein- oder zweifach durch A, OH, OA, Aryloxy mit 6-10 C-Atomen, Aralkyloxy mit 7-11 C-Atomen, -O-(CH₂)ₙ-O-, Hal, CF₃, NO₂, NH₂, NHA, NA₂, NHAc, NAAc, NHSO₂A und/oder NASO₂A substituierte Phenylreste,
- X: O, S, SO oder SO₂,
- m: 1, 2 oder 3,
- n: 1 oder 2
- A: einen Alkylrest mit 1-6 C-Atomen,
- Hal: F, Cl, Br oder I
und
- Ac: Alkanoyl mit 1-8 C-Atomen, Aralkanoyl mit 1-10 C-Atomen oder Aroyl mit 7-11 C-Atomen
bedeuten,
sowie deren pysiologisch unbedenkliche Salze.

Der Erfindung lag die Aufgabe zugrunde, neue Verbindungen aufzufinden, die zur Herstellung von Arzneimitteln verwendet werden können.

Es wurde gefunden, daß die genannten Substanzen bei guter Verträglichkeit wertvolle pharmakologische Eigenschaften besitzen. So zeigen sie z.B. das Zentralnervensystem beeinflussende, vorzugsweise dämpfende (z.B. sedierende, tranquillierende, neuroleptische und/oder antidepressive) Wirkungen. Im einzelnen haben die Verbindungen eine dämpfende Wirkung auf das Verhalten bei Mäusen (Methodik vgl. Irwin, Psychopharmacologica 13 (1968), 222-257). Sie hemmen bei Mäusen das durch Apomorphin induzierte Kletterverhalten (Methodik vgl. Costall et al., European J. Pharmacol. 50 (1968), 39-50) oder induzieren contralaterales Drehverhalten in Hemiparkinson-Ratten (feststellbar nach der Methode von Ungerstedt et al., Brain Res. 24 (1970), 485-493), ohne daß nennenswerte kataleptische Nebenwirkungen auftreten (Methodik vgl. Dolini-Stola, Pharmakopsychiat. 6 (1973), 189-197). Weiterhin hemmen die Substanzen die Bindung von tritiierten Dopaminagonisten und - antagonisten an striäre Rezeptoren (feststellbar nach der Methode von Schwarcz et al., J. Neurochemistry 34 (1980), 772-778, und Creese et al., European J. Pharmacol. 46 (1977), 377-381). Zusätzlich hemmen die Verbindungen den Zungen-Kieferreflex bei der narkotisierten Ratte (feststellbar in Anlehnung an die Methoden von Barnett et al., European J. Pharmacol. 21 (1973), 178-182, und von Ilhan et al., European J. Pharmacol 33 (1975), 61-64). Weiterhin treten analgetische und blutdrucksenkende Wirkungen auf; so wird der bei kathetertragenden wachen, spontan hypertonen Ratten (Stamm SHR/NIH-MO//CHB-EMD; Methode vgl. Weeks und Jones, Proc.Soc.Exptl.Biol.Med. 104 (1960), 646-648) direkt gemessene arterielle Blutdruck nach intragastraler Gabe der Verbindungen gesenkt.

Oxazolidinone mit Wirkungen auf das Zentralnervensystem sind z.B. beschrieben in EP 0 300 272, EP 0 443 197 und EP 0 459 256, ferner in Bioorganic and Medicinal Chemistry Letters, Bd. 2 (2), 165-170 (1992). Aroylpiperidine sind beschrieben in J. Med. Chem., Bd. 13 (1), 1-6 (1970).

Verbindungen der Formel I und ihre physiologisch unbedenklichen Säureadditionssalze können daher als Arzneimittelwirkstoffe verwendet werden.

Gegenstand der Erfindung sind die Piperidinderivate der Formel I sowie ihre Salze.

Gegenstand der Erfindung ist ferner ein Verfahren zur Herstellung von Piperidin-derivaten der Formel I sowie deren Salzen, dadurch gekennzeichnet, daß man eine Verbindung der Formel II 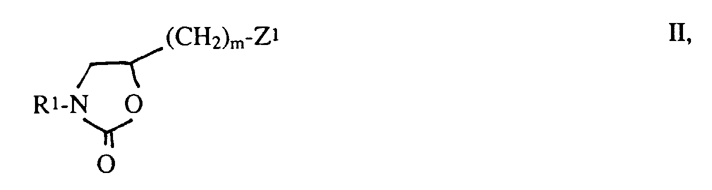 worin
- R¹ und m: die in Anspruch 1 angegebenen Bedeutungen haben und
- Z¹: Z oder NH₂,
- Z: Cl, Br, I, OH, SO₃CH₃ oder eine andere reaktionsfähige funktionell abgewandelte OH-Gruppe bedeuten,
sofern Z¹ = Z ist,
mit einer Verbindung der Formel III 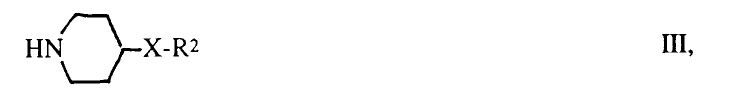 worin
- R² und X: die in Anspruch 1 angegebenen Bedeutungen haben
oder, sofern Z¹ = NH₂ ist,
mit einer Verbindung der Formel IIIa 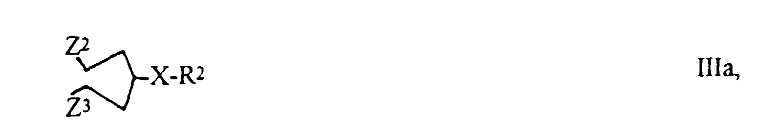 worin
- R² und X: die in Anspruch 1 angegebenen Bedeutungen haben und
- Z² und Z³: gleich oder verschieden sind und jeweils Cl, Br, I, OH, SO₃CH₃ oder eine andere reaktionsfähige funktionell abgewandelte OH-Gruppe bedeuten,
umsetzt,
daß man eine sonst der Formel I entsprechende Verbindung, die jedoch anstelle eines oder mehrerer Wasserstoffatome eine oder mehrere reduzierbare Gruppen und/oder eine oder mehrere zusätzliche -SO₂- und/oder -SO-Gruppen enthält, mit einem reduzierenden Mittel behandelt,
oder daß man zur Herstellung einer Verbindung der Formel I nach Anspruch 1 einen Rest R¹ und/oder R² in einen anderen Rest R¹ und/oder R² umwandelt,
oder daß man eine Verbindung der Formel IV 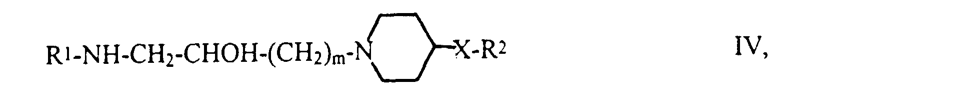 worin R¹, R², X und m die angegebenen Bedeutungen besitzen, mit einem geeigneten reaktionsfähigen Kohlensäurederivat umsetzt, oder daß man eine Verbindung der Formel V 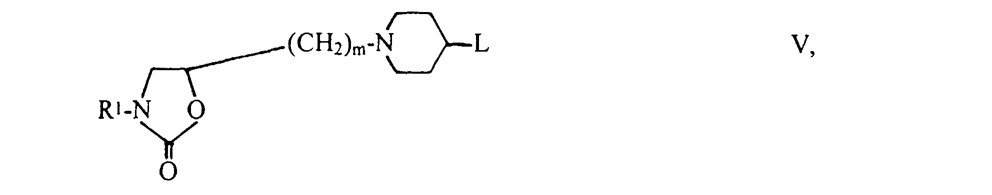 worin
- R¹ und m: die angegebenen Bedeutungen haben und
- L: Cl, Br oder eine andere geeignete Abgangsgruppe bedeutet,
mit einer Verbindung der Formel VI

X¹-R² VI,

worin
- R²: die angegebene Bedeutung hat und
- X¹: OH, SO₂H, SH bedeutet oder einen daraus ableitbaren geeigneten salzartigen Rest darstellt,
umsetzt,
und/oder daß man gegebenenfalls eine Verbindung der Formel I aus einem ihrer funktionellen Derivate durch Behandeln mit einem solvolysierenden oder hydrogenolysierenden Mittel in Freiheit setzt, oder eine Verbindung der Formel I durch Reduktion oder Oxidation in eine andere Verbindung der Formel I umwandelt, und/oder daß man eine Base der Formel I gemäß Anspruch 1 durch Behandeln mit einer Säure in eines ihrer Salze umwandelt.

Vor- und nachstehend haben die Reste R¹, R², A, Ac, X und Hal sowie die Parameter m und n die bei der Formel I angegebenen Bedeutungen, falls nicht ausdrücklich etwas anderes angegeben ist.

In den Formeln oder Teilformeln steht A für einen Alkylrest mit 1-6, vorzugsweise 1, 2, 3 oder 4 C-Atomen. A bedeutet bevorzugt Methyl, weiterhin Ethyl, Propyl, Isopropyl, Butyl, Isobutyl, sek.-Butyl oder tert.-Butyl, ferner auch Pentyl, 1-, 2- oder 3-Methylbutyl, 1,1- 1,2- oder 2,2-Dimethylpropyl, Hexyl, 1-, 2- oder 3-Methylpentyl.

Die Reste R¹ und R² können gleich oder verschieden sein. R¹ und R² bedeuten vorzugsweise, jeweils unabhängig voneinander, unsubstituiertes oder substituiertes Phenyl, wobei die Substituenten jeweils in ortho- oder meta-, besonders bevorzugt aber in para-Position des Phenylrestes stehen können.

Im einzelnen bedeuten R¹ und R² vorzugsweise Phenyl, p-Fluor-, p-Chlor-, p-Hydroxy-, p-Methoxy-, p-Nitro-, p-Methyl-, p-tert.-Butyl-, p-Phenylmethoxy- oder p-Acetamidophenyl bzw. p-N-Methylacetamidophenyl.

Ferner kann R¹ und R² auch bevorzugt 3,4-Methylendioxy-, p-Propionylami - oder p-Methylsulfonamidophenyl bedeuten.

Ac steht vorzugsweise für Acetyl oder Propionyl, ferner aber auch für Formyl, Butyryl, Isobutyryl, Valeryl, Isovaleryl, Pivaloyl (Trimethylacetyl), weiterhin bevorzugt für gegebenenfalls substituiertes Aroyl mit 7-11 C-Atomen, wobei als Substituenten vorzugsweise eine der folgenden Gruppen in Frage kommen: Alkyl, Alkoxy, Alkylthio, Alkylsulfinyl oder Alkylsulfonyl mit jeweils 1-3, vorzugsweise 1 oder 2 C-Atomen, Methylendioxy, ferner OH, F, Cl, Br, I, NO₂, NH₂, Alkylamino oder Diathylamino mit jeweils 1-3, vorzugsweise 1 oder 2 C-Atomen in der Alkylgruppe. Einzelne bevorzugte Aroylreste sind Benzoyl, o-, m- oder p-Toluyl, o-, m- oder p-Methoxybenzoyl, 2,3-, 2,4-, 2,5-, 2,6-, 3,4-, oder 3,5-Dimethoxybenzoyl, 2,3,4- 2,3,5-, 2,3,6-, 2,4,5-, 2,4,6- oder 3,4,5-Trimethoxybenzoyl, o-, m- oder p-Methylsulfonylbenzoyl, 2,3- oder 3,4-Methylendioxybenzoyl, 1- oder 2-Naphthoyl. Ac kann weiterhin für Aralkanoyl mit 1-10 C-Atomen wie z.B. Phenylacetyl, 2- oder 3-Phenylpropionyl oder 2-, 3- oder 4-Phenylbutyryl oder 2- oder 3-Phenylisobutyryl stehen.

X bedeutet bevorzugt Sauerstoff oder Schwefel, ferner aber auch vorzugsweise SO₂, während Hal vorzugsweise F oder Cl bedeutet.

Dementsprechend sind Gegenstand der Erfindung insbesondere diejenigen Verbindungen der Formel I, in denen mindestens einer der genannten Reste eine der angegebenen, insbesondere der angegebenen bevorzugten Bedeutungen hat.

Einige bevorzugte Gruppen von Verbindungen können durch die folgenden Teilformeln Ia bis If ausgedrückt werden, die der Formel I entsprechen und worin die nicht näher bezeichneten Reste und Parameter die bei der Formel I angegebene Bedeutung haben, worin jedoch
- in Ia: R¹ p-Methoxyphenyl oder Phenyl und R² p-Acetamidophenyl bedeuten;
- in Ib: R¹ p-h/iethoxyphenyl und R² Phenyl, m-Methoxy-, p-Methoxy-, p-Hydroxy-, p-Chlor-, p-Fluor-, p-Phenylmethoxy-, 3,4-Methylendioxy-, p-Methyl- oder p-tert.-Butylphenyl bedeuten;
- in Ic: R¹ p-Methoxyphenyl, X Sauerstoff und m = 1 bedeuten;
- in Id: R² p-Acetarnidophenyl, X Sauerstoff und m = 1 bedeuten;
- in Ie: R¹ p-Methoxyphenyl, R² p-Acetamido-, p-Methoxy-, p-Chlor-, p-Methyl-, p-tert.-Butyl- oder p-Methylsulfonamidophenyl und X Schwefel bedeuten;
- in If: R¹ p-Methoxyphenyl, R² p-Acetarnido-, p-Methoxy- oder p-Methylsulfonamidophenyl und X -SO₂- bedeuten.

Die Herstellung der Verbindungen der Formel I erfolgt im übrigen nach an sich bekannten Methoden, wie sie in der Literatur (z.B. in den Standardwerken wie Houben-Weyl, Methoden der Organischen Chemie, Georg-Thieme-Verlag; J. March, Advanced Organic Chemistry 3rd. Ed. (1985) oder Organic Reactions, beide John Wiley & Sons, Inc. New York) beschrieben sind, und zwar unter Reaktionsbedingungen, wie sie für die genannten Umsetzungen bekannt und geeignet sind. Dabei kann man auch von an sich bekannten, hier nicht näher erwähnten Varianten Gebrauch machen.

Die Ausgangsstoffe für das beanspruchte Verfahren können gewünschtenfalls auch in situ gebildet werden, derart, daß man sie aus dem Reaktionsgemisch nicht isoliert, sondern sofort weiter zu den Verbindungen der Formel I umsetzt.

In den Verbindungen der Formel II ist Z¹ vorzugsweise Z; dementsprechend werden die Verbindungen der Formel II insbesondere mit Piperidinderivaten der Formel III umgesetzt, um Verbindungen der Formel I zu erhalten. Der Rest Z ist vorzugsweise Cl oder Br; er kann jedoch auch I, OH oder eine reaktionsfähige funktionell abgewandelte OH-Gruppe bedeuten, insbesondere Alkylsulfonyloxy mit 1-6 (z.B. Methansulfonyloxy) oder Arylsulfonyloxy mit 6-10 C-Atomen (z.B. Benzolsulfonyloxy, p-Toluolsulfonyloxy, 1- oder 2-Naphthalin-sulfonyloxy).

Es ist jedoch auch möglich, daß Z¹ in Verbindungen der Formel II NH₂ bedeutet. Derartige Verbindungen werden dann mit Verbindungen der Formel IIIa, in denen Z² und Z³ gleich oder verschieden sein können und vorzugsweise Cl oder Br, ferner aber auch I, OH oder eine reaktionsfähige funktionell abgewandelte OH-Gruppe, vorzugsweise wie oben angegeben, bedeuten, umgesetzt.

Die Verbindungen der Formeln II, III und IIIa sind zum Teil bekannt; die nicht bekannten Verbindungen der Formeln II, III und IIIa können leicht analog zu den bekannten Verbindungen hergestellt werden. Primäre Alkohole der Formel II sind z.B. durch Reduktion der entsprechenden Carbonsäuren oder ihrer Ester erhältlich. Behandeln mit Thionylchlorid, Bromwasserstoff, Phosphortribromid oder ähnlichen Halogenverbindungen liefert die entsprechenen Halogenide der Formel II.

Die Sulfonyloxyverbindungen der Formel II sind aus den entsprechenden Alkoholen durch Umsetzung mit den entsprechenen Sulfonsäurechloriden erhältlich. Die Iodverbindungen der Formel II sind z.B. durch Einwirkung von Kaliumjodid auf die zugehörigen p-Toluolsulfonsäureester erhältlich. Die Amine der Formel II sind z.B. aus den Halogeniden mit Phthalimidkalium oder durch Reduktion der entsprechenden Nitrile herstellbar.

Die Piperidine der Formel III sind teilweise bekannt oder sind in Analogie zu den bekannten Verbindungen herstellbar. Man erhält sie beispielsweise durch Umsetzung von 4-Halogenpiperidinen mit geeigneten Phenolaten oder z.B. durch Reaktion von 4-Hydroxypiperidinen, wobei die Hydroxygruppe auch zu einer reaktionsfähigen Gruppe funktionell abgewandelt sein kann, mit entsprechenden Thiophenolen oder Thiophenolaten und gegebenenfalls anschließende Oxidation der S-Gruppe zu -SO- oder -SO₂-Gruppierungen. Verbindungen der Formel IIIa sind z.B. herstellbar durch Reduktion entsprechnder Diester zu Diolen und gegebenenfalls anschließende Umsetzung mit SOCl₂ bzw. PBr₃.

Die Umsetzung der Verbindungen II und III verläuft nach Methoden, wie sie für die Alkylierung von Aminen aus der Literatur bekannt sind. Man kann ohne Gegenwart eines Lösungsmittels die Komponenten miteinander verschmelzen, gegebenenfalls im geschlossenen Rohr oder im Autoklaven. Es ist aber auch möglich, die Verbindungen in Gegenwart eines indifferenten Lösungsmittels umzusetzen. Als Lösungsmittel eignen sich z.B. Kohlenwasserstoffe, wie Benzol, Toluol, Xylol; Ketone wie Aceton, Butanon; Alkohole wie Methanol, Ethanol, Isopropanol, n-Butanol; Ether wie Tetrahydrofuran (THF) oder Dioxan; Amide wie Dimethylformamid (DMF) oder N-Methyl-pyrrolidon; Nitrile wie Acetonitril, gegebenenfalls auch Gemische dieser Lösungsmittel untereinander oder Gemische mit Wasser. Der Zusatz eines säurebindenden Mittels, beispielsweise eines Alkali- oder Erdalkalimetallhydroxids, -carbonats oder -bicarbonats oder eines anderen Salzes einer schwachen Säure der Alkali- oder Erdalkalimetalle, vorzugsweise des Kaliums, Natriums oder Calciums, oder der Zusatz einer organischen Base wie Triethylamin; Dimethylanilin, Pyridin oder Chinolin oder eines Überschusses der Aminkomponente bzw. der Verbindung der Formel III oder IIIa kann günstig sein. Die Reaktionstemperatur liegt je nach den angewendeten Bedingungen zwischen etwa 0 und 150°, normalerweise zwischen 20 und 130°.

Es ist ferner möglich, eine Verbindung der Formel I zu erhalten, indem man ein Vorprodukt, das anstelle von Wasserstoffatomen eine oder mehrere reduzierbare Gruppe(n) und/oder eine oder mehrere zusätzliche C-C- und/oder C-N-Bindung(en) enthält, mit einem reduzierenden Mittel behandelt, vorzugsweise bei Temperaturen zwischen -80 und +250° in Gegenwart mindestens eines inerten Lösungsmittels.

Reduzierbare (durch Wasserstoff ersetzbare) Gruppen sind insbesondere Sauerstoff in einer Carbonylgruppe, Hydroxyl, Arylsulfonyloxy (z.B. p-Toluolsulfonyloxy), N-Benzolsulfonyl, N-Benzyl oder O-Benzyl.

Es ist grundsätzlich möglich, Verbindungen, die nur eine, oder solche, die nebeneinander zwei oder mehr dieser Gruppen bzw. zusätzlichen Bindungen enthalten, reduktiv in eine Verbindung der Formel I überzuführen. Vorzugsweise bedient man sich hierzu der katalytischen Hydrierung, des nascierenden Wasserstoffs oder bestimmter komplexer Metallhydride wie NaBH₄ oder LiAlH₄.

Für die katalytische Hydrierung sind als Katalysatoren beispielsweise Edelmetall-, Nickel- und Kobaltkatalysatoren geeignet. Die Edelmetallkatalysatoren können auf Trägern (z.B. Platin oder Palladium auf Kohle, Palladium auf Calciumcarbonat oder Strontiumcarbonat), als Oxidkatalysatoren (z.B. Platinoxid), oder als feinteilige Metallkatalysatoren vorliegen. Nickel- und Kobaltkatalysatoren werden zweckmäßig als Raney-Metalle, Nickel auch auf Kieselgur oder Bimsstein als Träger eingesetzt. Die Hydrierung kann bei Raumtemperatur und Normaldruck oder auch bei erhöhter Temperatur und/oder erhöhtem Druck durchgeführt werden. Vorzugsweise arbeitet man bei Drucken zwischen 1 und 100 at und bei Temperaturen zwischen -80 und +150°, in erster Linie zwischen Raumtemperatur und +100°. Die Umsetzung wird zweckmäßig im sauren, neutralen oder basischen Bereich und in Gegenwart eines Lösungsmittels, wie Wasser, Methanol, Ethanol, Isopropanol, n-Butanol, Ethylacetat, Dioxan, Essigsäure oder THF durchgeführt; man kann auch Gemische dieser Lösungsmittel untereinander anwenden.

Wird als Reduktionsmittel nascierender Wasserstoff verwendet, so kann man diesen z.B. durch Behandlung von Metallen mit schwachen Säuren oder mit Basen erzeugen. So kann man z.B. ein Gemisch von Zink mit Alkalilauge oder von Eisen mit Essigsäure verwenden. Geeignet ist auch die Verwendung von Natrium oder einem anderen Alkalimetall in einem Alkohol wie Ethanol, Isopropanol, Butanol, Amyl- oder Isoamylalkohol oder Phenol. Man kann ferner eine Aluminium-Nickel-Legierung in alkalisch-wäßriger Lösung, gegebenenfalls unter Zusatz von Ethanol verwenden. Auch Natrium- oder Aluminiumamalgam in wäßrig-alkoholischer oder wäßriger Lösung ist zur Erzeugung des nascierenden Wasserstoffs geeignet. Die Umsetzung kann auch in heterogener Phase durchgeführt werden, wobei man zweckmäßig eine wäßrige und eine Benzol- oder Toluol-Phase verwendet.

Als Reduktionsmittel können ferner komplexe Metallhydride, wie NaBH₄, Diisobutylaluminiumhydrid oder NaAl(OCH₂CH₂OCH₃)₂H₂ sowie Diboran eingesetzt werden, falls erwünscht unter Zusatz von Katalysatoren wie BF₃, AlCl₃ oder LiBr. Als Lösungsmittel eignen sich hierfür insbesondere Ether wie Diethylether, Di-n-butylether, THF, Dioxan, Diglyme oder 1,2-Dimethoxyethan sowie Kohlenwasserstoffe wie Benzol. Für eine Reduktion mit NaBH₄ sind in erster Linie Alkohole wie Methanol oder Ethanol, ferner Wasser sowie wäßrige Alkohole als Lösungsmittel geeignet. Nach diesen Methoden reduziert man vorzugsweise bei Temperaturen zwischen -80 und +150°, insbesondere zwischen 0 und etwa 100°.

Verbindungen der Formel I sind ferner erhältlich, indem man einen aromatischen Rest R¹ und/oder R² durch beispielsweise eine elektrophile Substitution in einen anderen Rest R¹ und/oder R² umwandelt.

Verbindungen der Formel I sind ferner durch Umsetzung von Aminoalkoholen der Formel IV mit reaktionsfähigen Derivaten der Kohlensäure erhältlich. Als solche eignen sich bevorzugt Dialkylcarbonate wie Dimethyl- oder Diethylcarbonat, Chlorameisensäureester wie Chlorameisensäuremethyl- oder -ethylester, N,N'-Carbonyldiimidazol oder Phosgen. Die Umsetzung gelingt zweckmäßig in Gegenwart eines inerten Lösungsmittels, vorzugsweise eine halogenierten Kohlenwasserstoffs wie Chloroform, eines Kohlenwasserstoffs wie Toluol oder eines Amids wie DMF bei Temperaturen zwischen etwa 20 und etwa 200, vorzugsweise zwischen 100 und 150°. Das Kohlensäurederivat wird zweckmäßig im Überschuß eingesetzt.

Außerdem können Verbindungen der Formel I erhalten werden, indem man Oxazolidinon-Derivate der Formel V, die ihrerseits beispielsweise durch Umsetzung von Verbindungen der Formel II (Z¹ = Z) mit entsprechenden Piperidin-Derivaten erhalten werden können, mit Verbindungen der Formel VI, unter Bedingungen, wie sie für die Ether- oder Thioetherbildung bekannt sind, umsetzt.

Die Verbindungen der Formel I können auch erhalten werden, indem man sie aus ihren funktionellen Derivaten durch Solvolyse, insbesondere Hydrolyse, oder durch Hydrogenolyse in Freiheit setzt.

Bevorzugte Ausgangsstoffe für die Solvolyse bzw. Hydrogenolyse sind solche, die sonst der Formel I entsprechen, aber anstelle einer oder mehrerer freier Amino- und/oder Hydroxygruppen entsprechende geschützte Amino- und/oder Hydroxygruppen enthalten, vorzugsweise solche, die anstelle eines H-Atoms, das mit einem N-Atom verbunden ist, eine Aminoschutzgruppe tragen, insbesondere solche, die anstelle einer HN-Gruppe eine R'-N-Gruppe tragen, worin R' eine Aminoschutzgruppe bedeutet, und/oder solche, die an Stelle des H-Atoms einer Hydroxygruppe eine Hydroxyschutzgruppe tragen, z.B. solche, die der Formel I entsprechen, jedoch an Stelle einer Gruppe -COOH eine Gruppe -COOR" tragen, worin R'' eine Hydroxyschutzgruppe bedeutet.

Es können auch mehrere - gleiche oder verschiedene - geschützte Amino- und/oder Hydroxygruppen im Molekül des Ausgangsstoffes vorhanden sein. Falls die vorhandenen Schutzgruppen voneinander verschieden sind, können sie in vielen Fällen selektiv abgespalten sein.

Der Ausdruck "Aminoschutzgruppe" ist allgemein bekannt und bezieht sich auf Gruppen, die geeignet sind, eine Aminogruppe vor chemischen Umsetzungen zu schützen (zu blockieren), die aber leicht entfernbar sind, nachdem die gewünschte chemische Reaktion an einer anderen Stelle des Moleküls durchgeführt worden ist. Typisch für solche Gruppen sind insbesondere unsubstituierte oder substituierte Acyl-, Aryl- (z.B. 2,4-Dinitrophenyl (DNP), Aralkoxymethyl- (z.B. Benzyloxymethyl (BOM)) oder Aralkyl-gruppen (z.B. Benzyl, 4-Nitrobenzyl, Triphenylmethyl). Da die Aminoschutzgruppen nach der gewünschten Reaktion (oder Reaktionsfolge) entfernt werden, ist ihre Art und Größe im übrigen nicht kritisch; bevorzugt werden jedoch solche mit 1-20, insbesondere 1-8 C-Atomen. Der Ausdruck "Acylgruppe" ist im Zusammenhang mit dem vorliegenden Verfahren im weitesten Sinne aufzufassen. Er umschließt von aliphatischen, araliphatischen, aromatischen oder heterocyclischen Carbonsäuren oder Sulfonsäuren abgeleitete Acylgruppen sowie insbesondere Alkoxycarbonyl-, Aryloxycarbonyl- und vor allem Aralkoxycarbonylgruppen. Beispiele für derartige Acylgruppen sind Alkanoyl wie Acetyl, Propionyl, Butyryl; Aralkanoyl wie Phenylacetyl; Aroyl wie Benzoyl oder Toluyl; Aryloxyalkanoyl wie Phenoxyacetyl; Alkoxycarbonyl wie Methoxycarbonyl, Ethoxycarbonyl, 2,2,2-Trichlorethoxycarbonyl, Isopropoxycarbonyl, tert.-Butoxycarbonyl (BOC), 2-Jodethoxycarbonyl; Aralkyloxycarbonyl wie Benzyloxycarbonyl (CBZ), 4-Methoxybenzyloxycarbonyl, 9-Fluorenylmethoxycarbonyl (FMOC). Bevorzugte Aminoschutzgruppen sind BOC, DNP und BOM, ferner CBZ, Benzyl und Acetyl.

Der Ausdruck "Hydroxyschutzgruppe" ist ebenfalls allgemein bekannt und bezieht sich auf Gruppen, die geeignet sind, eine Hydroxygruppe vor chemischen Umsetzungen zu schützen, die aber leicht entfernbar sind, nachdem die gewünschte chemische Reaktion an einer anderen Stelle des Moleküls durchgeführt worden ist. Typisch für solche Gruppen sind die oben genannten unsubstituierten oder substituierten Aryl-, Aralkyl- oder Acylgruppen, ferner auch Alkylgruppen. Die Natur und Größe der Hydroxyschutzgruppen ist nicht kritisch, da sie nach der gewünschten chemischen Reaktion oder Reaktionsfolge wieder entfernt werden; bevorzugt sind Gruppen mit 1-20, insbesondere 1-10 C-Atomen. Beispiele für Hydroxyschutzgruppen sind u.a. tert.-Butyl, Benzyl, p-Nitrobenzoyl, p-Toluolsulfonyl und Acetyl, wobei Benzyl und Acetyl besonders bevorzugt sind.

Das In-Freiheit-Setzen der Verbindungen der Formel I aus ihren funktionellen Derivaten gelingt -je nach der benutzten Schutzgruppe - z.B. mit starken Säuren, zweckmäßig mit Trifluoressigsäure oder Perchlorsäure, aber auch mit anderen starken anorganischen Säuren wie Salzsäure oder Schwefelsäure, starken Carbonsäuren wie Trichloressigsäure oder Sulfonsäuren wie Benzol- oder p-Toluolsulfonsäure. Die Anwesenheit eines zusätzlichen inerten Lösungsmittels ist möglich, aber nicht immer erforderlich.

Als inerte Lösungsmittel eignen sich vorzugsweise organische, beispielsweise Carbonsäuren wie Essigsäure, Ether wie Tetrahydrofuran oder Dioxan, Amide wie Dimethylformamid (DMF), halogenierte Kohlenwasserstoffe wie Dichlormethan, ferner auch Alkohole wie Methanol, Ethanol oder Isopropanol sowie Wasser. Ferner kommen Gemische der vorgenannten Lösungsmittel in Frage.

Trifluoressigsäure wird vorzugsweise im Überschuß ohne Zusatz eines weiteren Lösungsmittels verwendet, Perchlorsäure in Form eines Gemisches aus Essigsäure und 70%iger Perchlorsäure im Verhältnis 9:1. Die Reaktionstemperaturen für die Spaltung liegen zweckmäßig zwischen etwa 0 und etwa 50°; vorzugsweise arbeitet man zwischen 15 und 30° (Raumtemperatur).

Die BOC-Gruppe kann z.B. bevorzugt mit 40%iger Trifluoressigsäure in Dichlormethan oder mit etwa 3 bis 5 n HCl in Dioxan bei 15-60° abgespalten werden, die FMOC-Gruppe mit einer etwa 5-20%igen Lösung von Di methylamin, Diethylamin oder Piperidin in DMF bei 15-50°. Eine Abspaltung der DNP-Gruppe gelingt z.B. auch mit einer etwa 3-10%igen Lösung von 2-Mercaptoethanol in DMF/Wasser bei 15-30°.

Hydrogenolytisch entfernbare Schutzgruppen (z.B. BOM, CBZ oder Benzyl) können z.B. durch Behandeln mit Wasserstoff in Gegenwart eines Katalysators (z.B. Edelmetallkatalysators wie Palladium, zweckmäßig auf einem Träger wie Kohle) abgespalten werden. Als Lösungsmittel eignen sich dabei die oben angegebenen, insbesondere z.B. Alkohole wie Methanol oder Ethanol oder Amide wie DMF. Die Hydrogenolyse wird in der Regel bei Temperaturen zwischen 0 und 100° und Drucken zwischen 1 und 200 bar, bevorzugt bei 20-30° und 1-10 bar durchgeführt. Eine Hydrogenolyse der CBZ-Gruppe gelingt z.B. gut an 5-10%igem Pd-C in Methanol bei 20-30°.

Weiterhin kann man gegebenenfalls eine Verbindung der Formel I nach an sich bekannten Methoden in eine andere Verbindung der Formel I umwandeln.

So können Ether (O-Alkylderivate) gespalten werden, wobei die entsprechenden Hydroxyderivate entstehen. Z.B. kann man die Ether spalten durch Behandeln mit Dimethylsulfid-Bortribromid-Komplex, z.B. in Toluol, 1,2-Dichlorethan, THF oder Dimethylsulfoxid, durch Verschmelzen mit Pyridin- oder Anilinhydrohalogeniden, vorzugsweise Pyridinhydrochlorid, bei etwa 150-250°, mit HBr/Essigsäure oder mit Al-trihalogeniden in chlorierten Kohlenwasserstoffen wie 1,2-Dichlorethan.

Die Verbindungen der Formel I können ein Asymmetriezentrum besitzen. Sie können daher bei ihrer Herstellung als Racemate oder, falls optisch aktive Ausgangsstoffe verwendet werden, auch in optisch aktiver Form erhalten werden. Erhaltene Racemate können, falls erwünscht, nach an sich bekannten Methoden mechanisch oder chemisch in ihre optischen Antipoden getrennt werden. Vorzugsweise werden aus dem Racemat durch Umsetzung mit einem optisch aktiven Trennmittel Diastereomere gebildet. Als Trennmittel eignen sich z.B. optisch aktive Säuren, wie die D- und L-Formen von Weinsäure, Dibenzoylweinsäure, Diacetylweinsäure, Camphersulfonsäure, Mandelsäure, Äpfelsäure oder Milchsäure. Die verschiedenen Formen der Diasteromeren können in an sich bekannter Weise, z.B. durch fraktionierte Kristallisation, getrennt, und die optisch aktiven Verbindungen der Formel I können in an sich bekannter Weise aus den Diasteromeren in Freiheit gesetzt werden.

Eine erhaltene Base der Formel I kann mit einer Säure in das zugehörige Säureadditionssalz übergeführt werden. Für diese Umsetzung eignen sich bevorzugt Säuren, die physiologisch unbedenkliche Salze liefern. So können anorganische Säuren verwendet werden, z.B. Schwefelsäure, Halogenwasserstoffsäuren wie Chlorwasserstoffsäure oder Bromwasserstoffsäure, Phosphorsäuren wie Orthophosphorsäuren, Salpetersäure, Sulfaminsäure, ferner organische Säuren, im einzelnen aliphatische, alicyclische, araliphatishe, aromatische oder heterocyclische ein- oder mehrbasige Carbon-, Sulfon- oder Schwefelsäuren, wie Ameisensäure,
Essigsäure, Propionsäure, Pivalinsäure, Diethylessigsäure, Malonsäure, Bernsteinsäure, Pimelinsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und disulfonsäuren, Laurylschwefelsäure. Säureadditionssalze, die nicht physiologisch unbedenklich sind (z.B. Pikrate), können sich zur Isolierung und Aufreinigung von Basen der Formel I eignen.

Die freien Basen der Formel I können, falls gewünscht, aus ihren Salzen durch Behandlung mit starken Basen wie Natrium- oder Kaliumhydroxid, Natrium- oder Kaliumcarbonat in Freiheit gesetzt werden.

Gegenstand der Erfindung ist ferner die Verwendung der Verbindungen der Formel I und ihrer physiologisch unbedenklichen Salze zur Herstellung pharmazeutischer Zubereitungen, insbesondere auf nicht-chemischem Wege. Hierbei können sie zusammen mit mindestens einem Träger- oder Hilfsstoff und gegebenenfalls in Kombination mit einem oder mehreren weiteren Wirkstoff(en) in eine geeignete Dosierungsform gebracht werden.

Gegenstand der Erfindung sind ferner Mittel, insbesondere pharmazeutische Zubereitungen, enthaltend mindestens eine Verbindung der Formel I und/oder eines ihrer physiologisch unbedenklichen Salze. Diese Zubereitungen können als Arzneimittel in der Human- oder Veterinärmedizin eingesetzt werden. Als Trägersubstanzen kommen organische oder anorganische Stoffe in Frage, die sich für die enterale (z.B. orale), parenterale oder topische Applikation eignen und mit den neuen Verbindungen nicht reagieren, beispielsweise Wasser, pflanzliche Öle, Benzylalkohole, Polyethylenglykole, Gelatine, Kohlehydrate wie Lactose oder Stärke, Magnesiumstearat, Talk, Vaseline. Zur enteralen Applikation dienen insbesondere Tabletten, Dragees, Kapseln, Sirupe, Säfte, Tropfen oder Suppositorien, zur parenteralen Applikation Lösungen, vorzugsweise ölige oder wäßrige Lösungen, ferner Suspensionen, Emulsionen oder Implantate, für die topische Anwendung Salben, Cremes oder Puder. Die neuen Verbindungen können auch lyophilisiert und die erhaltenen Lyophilisate z.B. zur Herstellung von Injektionspräparaten verwendet werden.

Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und/oder Aromastoffe enthalten. Sie können, falls erwünscht, auch einen oder mehrere weitere Wirkstoffe enthalten, z.B. ein oder mehrere Vitamine.

Die Verbindungen der Formel I und ihre physiologisch unbedenklichen Salze können bei der therapeutischen Behandlung des menschlichen oder tierischen Körpers und bei der Bekämpfung von Krankheiten verwendet werden, insbesondere von Schizophrenie und psychoreaktiven Störungen und Psychopathien, von Depressionen, von schweren chronischen Schmerzen und von Krankheiten, die mit erhöhtem Blutdruck einhergehen. Weiterhin können die Verbindungen bei der Behandlung extrapyramidaler Störungen Anwendung finden. Die Verbindungen stellen gute atypische Neuroleptika dar und zeigen bei ihrer Anwendung keine nennenswerte kataleptische Nebenwirkungen.

Dabei werden die erfindungsgemäßen Substanzen in der Regel in Analogie zu bekannten, im Handel befindlichen Präparaten (Thioridazin, Haloperidol) verabreicht, vorzugsweise in Dosierungen zwischen etwa 0,2 und 500 mg, insbesondere zwischen 0,2 und 50 mg pro Dosierungseinheit. Die tägliche Dosierung liegt vorzugsweise zwischen etwa 0,003 und 10 mg/kg Körpergewicht.

Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der dungsgeschwindigkeit, Arzneistoffkombination und Schwere der jeweiligen Erkrankung, welcher die Therapie gilt. Die orale Applikation ist bevorzugt.

In nachstehenden Beispielen bedeutet "übliche Aufarbeitung": Man gibt, falls erforderlich, Wasser hinzu, extrahiert mit Dichlormethan, trennt ab, trocknet die organische Phase über Natriumsulfat, filtriert, dampft ein und reinigt durch Chromatographie an Kieselgel und/oder durch Kristallisation. Temperaturen sind in °C angegeben. Die [α]_{D}-Werte wurden bei 20° in Dimethylsulfoxid (DMSO) gemessen.

### Beispiel 1

Man kocht eine Lösung von 3,01 g 5-(Methansulfonyloxymethyl)-3-p-methoxyphenyl-oxazolidin-2-on ("A") [erhältlich durch Reaktion von 2,3-Epoxy-1-propanol mit N-Benzyl-p-methoxyanilin zu 1-N-Benzyl-p-methoxyanilinopropan-2,3-diol, Hydrogenolyse zu p-Methoxyanilino-propan-2,3-diol, Umsetzung mit Diethylcarbonat zu 5-(Hydroxymethyl5-(Hydroxymethyl)-3-p-methoxyphenyl-oxazolidin-2-on und Reaktion mit Methan-sulfonylchlorid], 1,58 g 4-(p-Acetamidophenoxy)-piperidin, 1,8 g Kaliumiodid und 1,4 g Kaliumcarbonat in 100 ml Acetonitril 12 Stunden, arbeitet nach Abkühlen wie üblich auf und erhält 3-p-Methoxyphenyl-5-[(4-p-acetamidophenoxy-piperidino)-methyl]oxazolidin-2-on.

### Analog erhält man durch Umsetzung von "A"

mit 4-(p-Methoxyphenoxy)-piperidin:
   3-p-Methoxyphenyl-5-[(4-p-methoxyphenoxy-piperidino)-methyl]-oxazolidin-2-on;
mit 4-(p-Chlorphenoxy)-piperidin:
   3-p-Methoxyphenyl-5-[(4-p-chlorphenoxy-piperidino)-methyl]-oxazolidin-2-on;
mit 4-(p-Fluorphenoxy)-piperidin:
   3-p-Methoxyphenyl-5-[(4-p-fluorphenoxy-piperidino)-methyl]-oxazolidin-2-on;
mit 4-(p-Phenylmethoxyphenoxy)-piperidin:
   3-p-Methoxyphenyl-5-[(4-p-phenylmethoxyphenoxy-piperidino)-methyl]-oxazolidin-2-on (Hydrochlorid);
mit 4-(p-Hydroxyphenoxy)-piperidin:
   3-p-Methoxyphenyl-5-[(4-p-hydroxyphenoxy-piperidino)-methyl]-oxazolidin-2-on; mit 4-(3,4-Methylendioxyphenoxy)-piperidin:
   3-p-Methoxyphenyl-5-[(4-(3,4-methylendioxyphenoxy)-piperidino)-methyl]-oxazolidin-2-on;
mit 4-(m-Methoxyphenoxy)-piperidin:
   3-p-Methoxyphenyl-5-[(4-m-methoxyphenoxy-piperidino)-methyl]-oxazolidin-2-on;
mit 4-Phenoxy-piperidin:
   3-p-Methoxyphenyl-5-[(4-phenoxy-piperidino)-methyl]-oxazolidin-2-on;
mit 4-(p-Nitrophenoxy)-piperidin:
   3-p-Methoxyphenyl-5-[(4-p-nitrophenoxy-piperidino)-methyl]-oxazolidin-2-on.

### Beispiel 2

Analog Beispiel 1 erhält man ausgehend von 5(R)-(Methansulfonyloxy-methyl)-3-p-methoxyphenyl-oxazolidin-2-on durch Umsetzung mit 4-(p-Acetamidophenoxy)-piperidin das 3-p-Methoxyphenyl-5(S)-[(4-p-acetamidophenoxy-piperidino)-methyl]-oxazolidin-2-on (Hydrochlorid), F. 195-196°; [α]_{D} = -28.8°(DMSO).

### Analog erhält man durch Umsetzung von 5(R)-(Methansulfonyloxymethyl)-3-p-methoxyphenyl-oxazolidin -2-on

mit 4-(p-Methoxyphenoxy)-piperidin:
   3-p-Methoxyphenyl-5(S)-[(4-p-methoxyphenoxy-piperidino)-methyl]-oxazolidin-2-on (Hydrochlorid), F. 249-250°; [α]_{D}= -27.8°(DMSO);
mit 4-(p-Chlorphenoxy)-piperidin:
   3-p-Methoxyphenyl-5(S)-[(4-p-chlorphenoxy-piperidino)-methyl]-oxazolidin-2-on (Hydrochlorid), F. 249-251°; [α]_{D}= -29.9°(DMSO);
mit 4-(p-Fluorphenoxy)-piperidin:
   3-p-Methoxyphenyl-5(S)-[(4-p-fluorphenoxy-piperidino)-methyl]-oxazolidin-2-on (Hydrochlorid), F. 246-247°; [α]_{D}= -28.9°(DMSO);
mit 4-(p-Phenylmethoxyphenoxy)-piperidin:
   3-p-Methoxyphenyl-5(S)-[(4-(p-phenylmethoxyphenoxy)-piperidino)-methyl]-oxazolidin-2-on (Hydrochlorid), F. 260-261°; [α]_{D}= -26.0°(DMSO);
mit 4-(p-Hydroxyphenoxy)-piperidin:
   3-p-Methoxyphenyl-5(S)-[(4p-hydroxyphenoxy-piperidino)-methyl]-oxazolidin-2-on (Hydrochlorid), F. 190-191°; [α]_{D}= -30.3°(DMSO);
mit 4-(3,4-Methylendioxyphenoxy)-piperidin:
   3-p-Methoxyphenyl-5(S)-[(4-(3,4-methylendioxyphenoxy)-piperidino)-methyl]-oxazolidin-2-on (Hydrochlorid), F. 227-229°; [α]_{D}= -28.9°(DMSO);
mit 4-(m-Methoxyphenoxy)-piperidin:
   3-p-Methoxyphenyl-5(S)-[(4-m-methoxyphenoxy-piperidino)-methyl]-oxazolidin-2-on (Hydrochlorid), F. 208-209°; [α]_{D}= -29.1°(DMSO);
mit 4-(p-Methansulfonylamidophenoxy)-piperidin:
   3-p-Methoxyphenyl-5(S)-[(4-p-methansulfonylamidophenoxy-piperidino)-methyl]-oxazolidin-2-on (Hydrochlorid), F. 184° (d); [α]_{D}= -31.6°(DMSO);
mit 4-Phenoxy-piperidin:
   3-p-Methoxyphenyl-5(S)-[(4-phenoxy-piperidino)-methyl]-oxazolidin-2-on(Hydrochlorid), F. 227-229°; [α]_{D}= -31.6°(DMSO);
mit 4-(p-Nitrophenoxy)-piperidin:
   3-p-Methoxyphenyl-5(S)-[(4-p-nitrophenoxy-piperidino)-methyl]-oxazolidin-2-on (Hydrochlorid), F. 250-253°; [α]_{D}= -32.7°(DMSO).

### Beispiel 3

Analog Beispiel 1 erhält man ausgehend von 5(R)-(Methansulfonyloxy-methyl)-3-p-methoxyphenyl-oxazolidin-2-on durch Umsetzung mit 4-(p-Acetamidophenylthio)-piperidin das 3-p-Methoxy-phenyl-5(S)-[(4-p-acetamidophenylthio-piperidino)-methyl]-oxazolidin-2-on, F. 178-179°; [α]_{D} = -27.6°(DMSO).

### Analog erhält man durch Umsetzung von 5(R)-(Methansulfonyloxy-methyl)-3-p-methoxyphenyl-oxazolidin-2-on

mit 4-(p-Methoxyphenylthio)-piperidin:
   3-p-Methoxyphenyl-5(S)-[(4-p-methoxyphenylthio-piperidino)-methyl]-oxazolidin-2-on (Hydrochlorid), F. 223-225°; [α]_{D}= -31.3°(DMSO);
mit 4-(p-Chlorphenylthio)-piperidin:
   3-p-Methoxyphenyl-5(S)-[(4-p-chlorphenylthio-piperidino)-methyl]-oxazolidin-2-on (Hydrochlorid), F. 233-236°; [α]_{D}= -30.8°(DMSO);
mit 4-(p-Methylphenylthio)-piperidin:
   3-p-Methoxyphenyl-5(S)-[(4-p-methylphenylthio-piperidino)-methyl]-oxazolidin-2-on (Hydrochlorid), F. 226-229°; [α]_{D}= -33.6°(DMSO);
mit 4-(p-tert.-Butylphenylthio)-piperidin:
   3-p-Methoxyphenyl-5(S)-[(4-(p-tert.-butylphenylthio)-piperidino)-methyl]-oxazolidin-2-on
   (Hydrochlorid), F. 231-234°; [α]_{D}= -30.9°(DMSO);
mit 4-(p-Methansulfonylamidophenylthio)-piperidin:
   3-p-Methoxyphenyl-5(S)-[(4-p-methansulfonylamidophenylthio-piperidino)-methyl]oxazolidin-2-on,
   F. 152-154°; [α]_{D}= -27.1°(DMSO).

### Beispiel 4

Eine Lösung von 1,2 g 3-p-Methoxyphenyl-5(S)-[(4-p-nitrophenoxy-piperidino)-methyl]-oxazolidin-2-on (F. 250-253°; erhältlich gemäß Bsp. 2) in 40 ml Methanol wird an 0,6 g Raney-Ni bei Raumtemperatur bis zum Stillstand der H₂-Aufnahme [p(H₂) = 1 bar] hydriert. Nach Filtration erhält man nach üblicher Aufarbeitung 3-p-Methoxyphenyl-5(S)-[(4-p-aminophenoxy-piperidino)-methyl]-oxazolidin-2-on, F. 236-240°, [α]_{D}-27,2° (DMSO).

### Beispiel 5

Eine Lösung von 1,4 g 3-p-Methoxyphenyl-5(S)-[(4-p-aminophenoxy-piperidino)-methyl]-oxazolidin-2-on und 2 ml Pyridin in 60 ml THF wird tropfenweise mit 1 ml Propionylchlorid versetzt und 2 Std. bei Raumtemperatur gerührt. Nach üblicher Aufarbeitung erhält man 3-p-Methoxyphenyl-5(S)-[(4-p-propionylaminophenoxy-piperidino)-methyl]-oxazolidin-2-on, F. 170 172°; [α]_{D}= -29.4°(DMSO).

### Analog erhält man durch Acylierung von

3-p-Methoxyphenyl-5(S)-[(4-p-aminophenoxy-piperidino)-methyl]-oxazolidin-2-on mit Acetylchlorid:
3-p-Methoxyphenyl-5(S)-[(4-p-acetamidophenoxy-piperidino)-methyl]-oxazolidin-2-on (Hydrochlorid), F. 195-196°; [α]_{D}= -28.8°(DMSO).

### Beispiel 6

Eine Lösung von 2,8 g 3-p-Methoxyphenyl-5(S)-[(4-p-acetamidophenoxy-piperidino)-methyl]-oxazolidin-2-on (F. 195-196°) in 40 ml Dimethylformamid wird unter Eiskühlung mit 0,5 g NaH und 1,7 ml Ethyliodid versetzt und 1 Stunde gerührt. Anschließend rührt man weitere 2 Stunden bei Raumtemperatur und erhält nach üblicher Aufarbeitung 3-p-Methoxyphenyl-5-[(4-(p-N-ethyl-acetamidophenoxy)-piperidino)-methyl]-oxazolidin-2-on.

### Analog erhält man durch Alkylierung des sekundären N-Atoms der entsprechenden Verbindung der Formel I:

aus 3-p-Methoxyphenyl-5(S)-[(4-p-acetamidophenoxy-piperidino)-methyl]-oxazolidin-2-on mit Methyliodid:
3-p-Methoxyphenyl-5(S)-[(4-(p-N-methyl-acetamidophenoxy)-piperidino)-methyl]-oxazolidin-2-on (Hydrochlorid), F. 254-257°, [α]_{D}-28,4° (DMSO).

### Beispiel 7

Analog Beispiel 1 erhält man ausgehend von 5(R)-(Methansulfonyloxymethyl)-3-phenyl-oxazolidin-2-on durch Umsetzung mit 4-(p-Acetamido-phenoxy)-piperidin das 3-Phenyl-5(S)-[(4-p-acetamidophenoxy-piperidino)-methyl]-oxazolidin-2-on (Hydrochlorid), F. > 260°; [α]_{D} = -27.1°(DMSO).

Analog erhält man durch Umsetzung von 4-(p-Acetamidophenoxy)-piperidin
mit 5(S)-(Methansulfonyloxymethyl)-3-phenyl-oxazolidin-2-on:
   3-Phenyl-5(R)-[(4-p-acetamidophenoxy-piperidino)-methyl]-oxazolidin-2-on (Hydrochlorid), F. > 260°;
mit 5(S)-(Methansulfonyloxymethyl)-3-p-chlorphenyl-oxazolidin-2-on:
   3-p-Chlorphenyl-5(R)-[(4-p-acetamidophenoxy-piperidino)-methyl]-oxazolidin-2-on (Hydrochlorid), F. 264-266°;
mit 5(R)-(Methansulfonyloxymethyl)-3-p-chlorphenyl-oxazolidin-2-on:
   3-p-Chlorphenyl-5(S)-[(4-p-acetamidophenoxy-piperidino)-methyl]-oxazolidin-2-on (Hydrochlorid), F. 264-266°; [α]_{D} = -31,7° (DMSO);
mit 5(S)-(Methansulfonyloxymethyl)-3-p-(phenylmethoxy)-phenyl-oxazolidin-2-on:
   3-p-(Phenylmethoxy)-phenyl-5(R)-[(4-p-acetamidophenoxy-piperidino)-methyl]-oxazolidin-2-on (Hydrochloridhydrat); F. 189-191°;
mit 5(R)-(Methansulfonyloxymethyl)-3-p-(phenylmethoxy)-phenyl-oxazolidin-2-on:
   3-p-(Phenylmethoxy)-phenyl-5(S)-[(4-p-acetamidophenoxy-piperidino)-methyl]-oxazolidin-2-on
   (Hydrochloridhydrat), F. 189-191°; [α]_{D} = -22.7° (DMSO);
mit 5(S)-(Methansulfonyloxymethyl)-3-p-hydroxyphenyl-oxazolidin-2-on:
   3-p-Hydroxyphenyl-5(R)-[(4-p-acetamidophenoxy-piperidino)-methyl]-oxazolidin-2-on (Hydrochlorid), F. 282-284°;
mit 5(R)-(Methansulfonyloxymethyl)-3-p-hydroxyphenyl-oxazolidin-2-on:
   3-p-Hydroxyphenyl-5(S)-[(4-p-acetamidophenoxy-piperidino)-methyl]-oxazolidin-2-on (Hydrochlorid), F. 282-284°; [α]_{D} = -25.5° (DMSO);
mit 5(S)-(Methansulfonyloxymethyl)-3-p-fluorphenyl-oxazolidin-2-on:
   3-p-Fluorphenyl-5(R)-[(4-p-acetamidophenoxy-piperidino)-methyl]-oxazolidin-2-on (Hydrochlorid), F. 267-268°;
mit 5(R)-(Methansulfonyloxymethyl)-3-p-fluorphenyl-oxazolidin-2-on:
   3-p-Fluorphenyl-5(S)-[(4-p-acetamidophenoxy-piperidino)-methyl]-oxazolidin-2-on(Hydrochlorid), F. 267-268°; [α]_{D} = -25.5° (DMSO);
mit 5(S)-(Methansulfonyloxymethyl)-3-p-methoxyphenyl-oxazolidin-2-on:
   3-p-Methoxyphenyl-5(R)-[(4-p-acetamidophenoxy-piperidino)-methyl]-oxazolidin-2-on (Hydrochlorid), F. 206-208°; [α]_{D} = +29.9° (DMSO).

### Beispiel 8

Analog Beispiel 1 erhält man ausgehend von 5-(2-Metbansulfonyloxyethyl)-3-p-methoxyphenyl-oxazolidin-2-on durch Umsetzung mit 4-(p-Acetamidophenoxy)-piperidin das 3-p-Methoxyphenyl-5-[2-(4-p-acetamidophenoxy-piperidino)-ethyl]-oxazolidin-2-on, F. 226-229°.

### Analog erhält man durch Umsetzung von 5-(2-Methansulfonyloxyethyl)-3-p-methoxyphenyl-oxazolidin-2-on

mit 4-(p-Methoxyphenoxy)-piperidin:
   3-p-Methoxlphenyl-5-[2-(4-p-methoxyphenoxy-piperidino)-ethyl]-oxazolidin-2-on;
mit 4-(p-Chlorphenoxy)-piperidin:
   3-p-Methoxlphenyl-5-[2-(4-p-chlorphenoxy-pipehdino)-ethyl]-oxazolidin-2-on;
mit 4-(p-Fluorphenoxy)-piperidin:
   3-p-Methoxyphenyl-5-[2-(4-p-fluorphenoxy-piperidino)-ethyl]-oxazolidin-2-on;
mit 4-(p-Phenylmethoxyphenoxy)-piperidin:
   3-p-Methoxyphenyl-5-[2-(4-p-phenylmethoxyphenoxy-piperidino)-ethyl]-oxazolidin-2-on;
mit 4-(p-Hydroxyphenoxy)-piperidin:
   3-p-Methoxyphenyl-5-[2-(4-p-hydroxyphenoxy-piperidino)-ethyl]-oxazolidin-2-on;
mit 4-(3,4-Methylendioxyphenoxy)-piperidin:
   3 -p-Methoxyphenyl-5-[2-(4-(3,4-methylendioxyphenoxy)-piperidino)-ethyl]-oxazolidin-2-on;
mit 4-(m-Methoxyphenoxy)-piperidin:
   3-p-Methoxyphenyl-5-[2-(4-m-methoxyphenoxy-piperidino)-ethyl]-oxazolidin-2-on;
mit 4-Phenoxy-piperidin:
   3-p-Methoxyphenyl-5-[2-(4-phenoxy-piperidino)-ethyl]-oxazolidin-2-on;
mit 4-(p-Nitrophenoxy)-piperidin:
   3-p-Methoxyphenyl-5-[2-(4-p-nitrophenoxy-piperidino)-ethyl]-oxazolidin-2-on;

### Analog erhält man durch Umsetzung von 5-(3-Methansulfonyloxypropyl)-3-p-methoxyphenyl-oxazolidin-2-on

mit 4-(p-Acetamidophenoxy)-piperidin:
   3 -p-Methoxyphenyl-5-[3 (4-p-acetimidophenoxy-piperidino)-propyl]-oxazolidin-2-on (Dihydrochlorid), F. 166-168°;
mit 4-(p-Methoxyphenoxy)-piperidin:
   3 -p-Methoxyphenyl-5-[3-(4-p-methoxyphenoxy-piperidino)-propyl]-oxazolidin-2-on (Hydrochlorid);
mit 4-(p-Chlorphenoxy)-piperidin:
   3-p-Methoxyphenyl-5-[3 (4-p-chlorphenoxy-piperidino)-propyl]-oxazolidin-2-on;
mit 4-(p-Fluorphenoxy)-piperidin:
   3-p-Methoxyphenyl-5-[3-(4-p-fluorphenoxy-piperidino)-propyl]-oxazolidin-2-on;
mit 4-(p-Phenylmethoxyphenoxy)-piperidin:
   3-p-Methoxyphenyl-5-[3-(4-p-phenyhnethoxyphenoxy-pipehdino)-propyl]-oxazolidin-2-on;
mit 4-(p-Hydroxyphenoxy)-piperidin:
   3-p-Methoxyphenyl-5-[3-(4-p-hydroxyphenoxy-piperidino)-propyl]-oxazolidin-2-on;
mit 4-(3,4-Methylendioxyphenoxy)-piperidin:
   3-p-Methoxyphenyl-5-[3-(4-(3,4-methylendioxyphenoxy)-piperidino)-propyl]-oxazolidin-2-on;
mit 4-(m-Methoxyphenoxy)-piperidin:
   3-p-Methoxyphenyl-5-[3--(4-m-methoxyphenoxy-piperidino)-propyl]-oxazolidin-2-on;
mit 4-Phenoxy-piperidin:
   3-p-Methoxyphenyl-5-[3-(4-phenoxy-piperidino)-propyl]-oxazolidin-2-on;
mit 4-(p-Nitrophenoxy)-piperidin:
   3 -p-Methoxyphenyl-5-[3-(4-p-nitrophenoxy-piperidino)-propyl]-oxazolidin-2-on.

### Beispiel 9

### Analog Beispiel 6 erhält man durch Alkylierung des sekundären N-Atoms der folgenden Verbindungen der Formel 1:

aus 3-Phenyl-5(R)-[(4-p-acetamidophenoxy-piperidino)-methyl]-oxazolidin-2-on:
   3-Phenyl-5(R)-[(4-p-(N-methyl-acetamidophenoxy)-piperidino)-methyl]-oxazolidin-2-on;
aus 3-p-Chlorphenyl-5(R)-[(4-p-acetamidophenoxy-piperidino)-methyl]-oxazolidin-2-on:
   3-p-Chlorphenyl-5(R)-[(4-p-(N-methyl-acetamidophenoxy)-piperidino)-methyl]-oxazolidin-2-on;
aus 3-p-Chlorphenyl-5(S)-[(4-p-acetamidophenoxy-piperidino)-methyl]-oxazolidin-2-on:
   3-p-Chlorphenyl-5(S)-[(4-p-(N-methyl-acetamidophenoxy)-piperidino)-methyl]-oxazolidin-2-on;
aus 3-p-(Phenylmethoxy)-phenyl-5(R)-[(4-p-acetamidophenoxy-piperidino)-methyl]-oxazolidin-2-on:
   3-p-(Phenylmethoxy)-phenyl-5(R)-[(4-p-(N-methyl-acetamidophen-oxy)-piperidino)-methyl]-oxazolidin-2-on;
aus 3-p-(Phenylmethoxy)-phenyl-5(S)-[(4-p-acetamidophenoxy-piperidi-no)-methyl]-oxazolidin-2-on:
   3-p-(Phenylmethoxy)-phenyl-5(S)-[(4-p-(N-methyl-acetamidophen-oxy)-piperidino)-methyl]-oxazolidin-2-on;
aus 3-p-Hydroxyphenyl-5(R)-[(4-p-acetamidophenoxy-piperidino)-methyl]-oxazolidin-2-on:
   3-p-Methoxyphenyl-5(R)-[(4-p-(N-methyl-acetamidophenoxy)-piperidino)-methyl]-oxazolidin-2-on;
aus 3-p-Hydroxyphenyl-5(S)-[(4-p-acetamidophenoxy-piperidino)-methyl]-oxazolidin-2-on:
   3-p-Methoxyphenyl-5(S)-[(4-p-(N-methyl-acetamidophenoxy)-piperidino)-methyl]-oxazolidin-2-on;
aus 3-p-Fluorphenyl-5(R)-[(4-p-acetamidophenoxy-piperidino)-methyl]-oxazolidin-2-on:
   3-p-Fluorphenyl-5(R)-[(4-p-(N-methyl-acetamidophenoxy)-piperidino)-methyl]-oxazolidin-2-on;
aus 3-p-Fluorphenyl-5(S)-[(4-p-acetamidophenoxy-piperidino)-methyl]-oxazolidin-2-on:
   3-p-Fluorphenyl-5(S)-[(4-p-(N-methyl-acetamidophenoxy)-piperidino)-methyl]-oxazolidin-2-on;
aus 3-p-Methoxyphenyl-5(R)-[(4-p-acetamidophenoxy-piperidino)-methyl]-oxazolidin-2-on:
   3-p-Methoxyphenyl-5(R)-[(4-p-(N-methyl-acetamidophenoxy)-piperidino)-methyl]-oxazolidin-2-on.

### Beispiel 10

Eine Lösung von 1,2 g 3-p-Methoxyphenyl-5(S)-[(4-p-acetamidophenyl-thiopiperidino)-methyl ]-oxazolidin-2-on (F. 178-179°) und 10 Äquivalenten Wasserstoffperoxid-Lösung (30%ig) in 100 ml Methanol wird 3 Stunden auf 60° erwärmt. Nach Eindampfen und üblicher Aufarbeitung erhält man 3-p-Methoxyphenyl-5(S)-[(4-p-acetamidophenylsulfonyl-piperidino)-methyl]-oxazolidin-2-on, F. 174-176°; [α]_{D}= = -24.0°(DMSO).

### Analog erhält man durch Oxidation der entsprechenden Thiopiperidinderivate:

aus 3-p-Methoxyphenyl-5(S)-[(4-p-methoxyphenylthio-piperidino)-methyl]-oxazolidin-2-on (Hydrochlorid; F. 223-225°):
   3-p-Methoxyphenyl-5(S)-[(4-p-methoxyphenylsulfonyl-piperidino)-methyl]-oxazolidin-2-on (Hydrochlorid), F. 254-256°; [α]_{D}= -31.6°(DMSO);
aus 3-p-Methoxyphenyl-5(S)-[(4-p-chlorphenylthio-piperidino)-methyl]-oxazolidin-2-on (Hydrochlorid; F. 233-236°):
   3-p-Methoxyphenyl-5(S)-[(4-p-chlorphenylsulfonyl-piperidino)-methyl]-oxazolidin-2-on;
aus 3-p-Methoxyphenyl-5(S)-[(4-p-methylphenylthio-piperidino)-methyl]-oxazolidin-2-on (Hydrochlorid; F. 226-229°):
   3-p-Methoxyphenyl-5(S)-[(4-p-methylphenylsulfonyl-piperidino)-methyl]-oxazolidin-2-on;
aus 3-p-Methoxyphenyl-5(S)-[(4-(p-tert.-butylphenylthio)-piperidino)-methyl]-oxazolidin-2-on (Hydrochlorid; F. 231-234°):
   3-p-Methoxyphenyl-5(S)-[(4-(p-tert.-butylphenylsulfonyl)-piperidino)-methyl]-oxazolidin-2-on; methyl]-oxazolidin-2-on;
aus 3-p-Methoxyphenyl-5(S)-[(4-p-methansulfonylamidophenylthio-piperidino)-methyl]-oxazolidin-2-on (F. 152-154°):
   3-p-Methoxyphenyl-5(S)-[(4-p-methansulfonylarnidophenylsulfonyl-piperidino)-methyl]-oxazolidin-2-on, F. 187-189°; [α]_{D} = -23.2°(DMSO).

### Beispiel 11

Eine Lösung von 1,6 g 3-p-Methoxyphenyl-5(S)-[(4-p-aminophenoxypiperidino)-methyl]-oxazolidin-2-on [erhältlich nach Beispiel 4] und 0,9 g Methansulfonylchlorid (gelöst in 5 ml THF) in 100 ml THF wird 3 Stunden bei Raumtemperatur gerührt. Nach Eindampfen und üblicher Aufarbeitung erhält man 3-p-Methoxyphenyl-5(S)-[(4-p-methansulfonyl-aminophenoxy-piperidino)-methyl]-oxazolidin-2-on (Hydrochlorid), F. 184° (d); [α]_{D} = -26,5° (DMSO).

Analog erhält man ausgehend von 3-p-Methoxyphenyl-5-[2-(4-p-aminophenoxy-piperidino)-ethyl]-oxazolidin-2-on das 3-p-Methoxyphenyl-5-[2-(4-p-methansulfonylaminophenoxy-piperidino)-ethyl]-oxazolidin-2-on.

### Beispiel 12

Analog Beispiel 1 erhält man ausgehend von 5(R)-(Methansulfonyloxymethyl)-3-p-methoxyphenyl-2-oxazolidinon durch Umsetzung mit 4-(3,4-Ethylendioxy-phenoxy)-piperidin das 3-p-Methoxyphenyl-5(s)-[(4-(3,4-ethylendioxy-phenoxypiperidino)-methyl]-2-oxazolidinon (Hydrochlorid), F. 219-221°; [α]_{D} = -28,5° (DMSO).

### Analog erhält man durch Umsetzung von 5(R)-(Methansulfonyloxymethyl)-3-p-methoxyphenyl-2-oxazolidinon

mit 4-(o-Acetamido-phenoxy)-piperidin:
   3-p-Methoxyphenyl-5(S)-[ (4-o-acetamido-phenoxypiperidino)-methyl]-2-oxazolidinon, F. 98-102°; [α]_{D} = -22,5° (DMSO);
mit 4-(m-Acetamido-phenoxy)-piperidin:
   3-p-Methoxyphenyl-5(S)-[(4-m-acetamido-phenoxypiperidino)-methyl]-2-oxazolidinon, F. 164-165°; [α]_{D} = -30,5° (DMSO);
mit 4-(p-Formamido-phenoxy)-piperidin:
   3-p-Methoxyphenyl-5(S)-[(4-p-formamido-phenoxypiperidino)-methyl]-2-oxazolidinon, F. 102-103°; [α]_{D} = -31,2° (DMSO);
mit 4-(p-Valerylamino-phenoxy)-piperidin:
   3-p-Methoxyphenyl-5(S)-[(4-p-valerylamino-phenoxypiperidino)-methyl]-2-oxazolidinon
   (Hydrochlorid), F. 224-226°;
   [α]_{D} = -26,7° (DMSO);
mit 4-(p-Isobutyrylamino-phenoxy)-piperidin:
   3-p-Methoxyphenyl-5(S)-[(4-p-isobutyrylamino-phenoxypiperidino)-methyl]-2-oxazolidinon (Hydrochlorid), F. 235-236°; [α]_{D} = -28,6° (DMSO).

### Beispiel 13

Analog Beispiel 1 erhalt man ausgehend von 5(R)-(Methansulfonyloxymethyl)-3-p-hydroxyphenyl-2-oxazolidinon durch Umsetzung mit 4-(p-Hydroxy-phenoxy)-piperidin das 3-p-Hydroxyphenyl-5(S)-[(4-p-hydroxy-phenoxypiperidino)-methyl]-2-oxazolidinon (Hydrochlorid), F. 241-245°; [α]_{D} = -28,0° (DMSO).

### Analog erhält man durch Umsetzung von 5(R)-(Methansulfonyloxymethyl)-3-p-hydroxyphenyl-2-oxazolidinon

mit 4-(p-Propionylamino-phenoxy)piperidin:
3-p-Hydroxyphenyl-5(S)-[(4-p-propionylaminophenoxypiperidino)-methyl]-2-oxazolidinon,
F. 122-125°; [α]_{D} = -25,0°(DMSO).

Die nachstehenden Beispiele betreffen pharmazeutische Zubereitungen.

### Beispiel A: Injektionsgläser

Eine Lösung von 100 g eines Wirkstoffes der Formel I und 5 g Dinatriumhydrogenphosphat in 3 l zweifach destilliertem Wasser wird mit 2 n Salzsäure auf pH 6,5 eingestellt, steril filtriert, in Injektionsgläser abgefüllt, lyophilisiert und steril verschlossen. Jedes Injektionsglas enthält 5 mg Wirkstoff.

### Beispiel B: Suppositorien

Man schmilzt ein Gemisch von 20 mg eines Wirkstoffes der Formel I mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines Wirkstoffes der Formel I, 9,38 g NaH₂PO₄· 2 H₂O, 28,48 g Na₂HPO₄· 12 H₂O und 0,1 g Benzalkoniumchlorid in 940 ml zweifach destilliertem Wasser. Man stellt auf pH 6,8 ein, füllt auf 1 l auf und sterilisiert durch Bestrahlung. Diese Lösung kann in Form von Augentropfen verwendet werden.

### Beispiel D: Salbe

Man mischt 500 mg eines Wirkstoffes der Formel I mit 99,5 g Vaseline unter aseptischen Bedinungen.

### Beispiel E: Tabletten

Ein Gemisch von 1 kg Wirkstoff der Formel I, 4 kg Lactose, 1,2 kg Kartoffelstärke, 0,2 kg Talk und 0,1 kg Magnesiumstearat wird in üblicher Weise zu Tabletten verpreßt, derart, daßjede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff der Formel I werden in üblicher Weise in Hartgelatinekapseln gefüllt, so daß jede Kapsel 20 mg des Wirkstoffs enthält.

### Beispiel H: Ampullen

Eine Lösung von 1 kg Wirkstoff der Formel I in 60 l zweifach destilliertem Wasser wird in Ampullen abgefüllt, unter aseptischen Bedingungen lyophilisiert und steril verschlossen. Jede Ampulle enthält 10 mg Wirkstoff.

## Patentansprüche

1. 4-Aryloxy- oder 4-Arylthiopiperidinderivate der Formel I 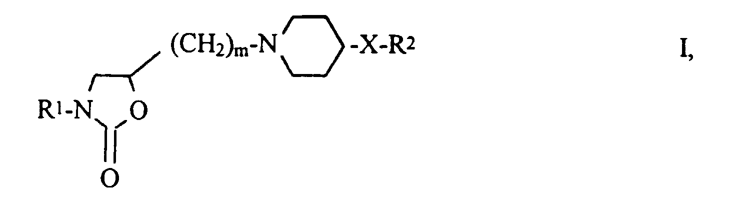 worin
R¹ und R² jeweils unabhängig voneinander unsubstituierte oder ein- oder zweifach durch A, OH, OA, Aryloxy mit 6-10 C-Atomen, Aralkyloxy mit 7-11 C-Atomen, -O-(CH₂)ₙ-O-, Hal, CF₃, NO₂, NH₂, NHA, NA₂, NHAc, NAAc, NHSO₂A und/oder NASO₂A substituierte Phenylreste,
X O, S, SO oder SO₂,
m 1, 2 oder 3,
n 1 oder 2
A einen Alkylrest mit 1-6 C-Atomen,
Hal F, Cl, Br oder I
und
Ac Alkanoyl mit 1-8 C-Atomen, Aralkanoyl mit 1-10 C-Atomen oder Aroyl mit 7-11 C-Atomen
bedeuten
sowie deren physiologisch unbedenkliche Salze.

2. Ein Enantiomer einer Verbindung der Formel I gemäß Anspruch 1.

3. (a) 3-p-Chlorphenyl-5- [(4-p-acetamidophenoxypipendino)-methyl]-oxazolidin-2-on;
(b) 3-p-Hydroxyphenyl-5-[(4-p-acetamidophenoxypiperidino)-methyl]-oxazolidin-2-on;
(c) 3-p-Methoxyphenyl-5-[(4-p-chlorphenoxypiperidino)-methyl]-oxazolidin-2-on;
(d) 3-p-Methoxyphenyl-5-[(4-p-hydroxyphenoxypiperidino)-methyl]-oxazolidin-2-on;
(e) 3-p-Methoxyphenyl-5-[(4-p-nitrophenoxypiperidino)-methyl]-oxazolidin-2-on;
(f) 3-p-Methoxyphenyl-5-[(4-p-chlorphenylthiopiperidino)-methyl]-oxazolidin-2-on;
(g) 3-p-Methoxyphenyl-5-[(4-p-methylphenylthiopiperidino)-methyl]-oxazolidin-2-on;
(h) 3-p-Methoxyphenyl-5-[(4-p-methoxyphenylsulfonyl-piperidino)-methyl]-oxazolidin-2-on.
gemäß Anspruch 1.

4. Verfahren zur Herstellung von Verbindungen der Formel I gemäß Anspruch 1 sowie von deren Salzen, dadurch gekennzeichnet, daß man eine Verbindung der Formel II 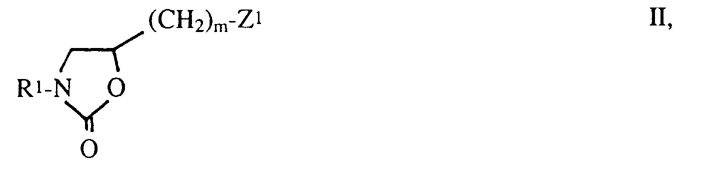 worin
R¹ und m die in Anspruch 1 angegebenen Bedeutungen haben und
Z¹ Z oder NH₂,
Z Cl, Br, I, OH, SO₃CH₃ oder eine andere reaktionsfähige funktionell abgewandelte OH-Gruppe bedeuten,
sofern Z¹ = Z ist,
mit einer Verbindung der Formel III 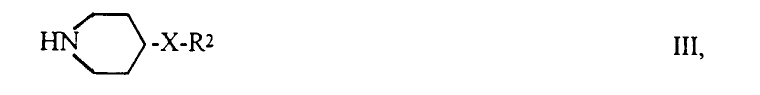 worin
R² und X die in Anspruch 1 angegebenen Bedeutungen haben
oder, sofern Z¹ = NH₂ ist,
mit einer Verbindung der Formel IIIa 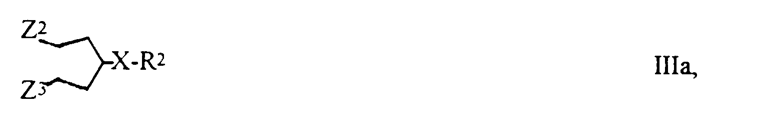 worin
R² und X die in Anspruch 1 angegebenen Bedeutungen haben und
Z² und Z³ gleich oder verschieden sind und jeweils Cl, Br, I, OH, SO₃CH₃ oder eine andere reaktionsfähige funktionell abgewandelte OH-Gruppe bedeuten,
umsetzt,
oder daß man eine sonst der Formel I entsprechende Verbindung, die jedoch anstelle eines oder mehrerer Wasserstoffatome eine oder mehrere reduzierbare Gruppen und/oder eine oder mehrere zusätzliche -SO₂- und/oder -SO-Gruppen enthält, mit einem reduzierenden Mittel behandelt,
oder daß man zur Herstellung einer Verbindung der Formel I nach Anspruch 1 einen Rest R¹ und/oder R² in einen anderen Rest R¹ und/oder R² umwandelt,
oder daß man eine Verbindung der Formel IV 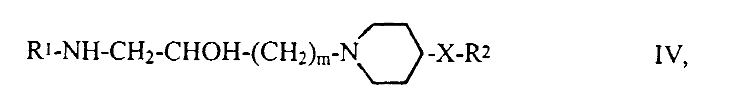 worin R¹, R², X und m die angegebenen Bedeutungen besitzen, mit einem geeigneten reaktionsfähigen Kohlensäurederivat umsetzt,
oder daß man eine Verbindung der Formel V 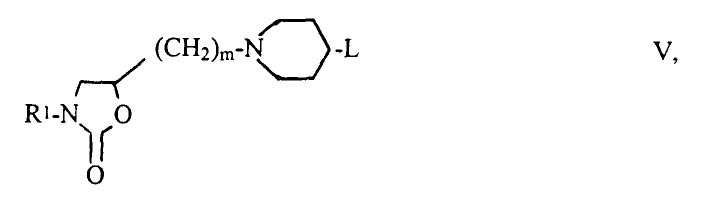 worin
R¹ und m die angegebenen Bedeutungen haben und
L Cl, Br oder eine andere geeignete Abgangsgruppe bedeutet,
mit einer Verbindung der Formel VI
X¹-R² VI,
worin
R² die angegebene Bedeutung hat und
X¹ OH, SO₂H, SH bedeutet oder einen daraus ableitbaren geeigneten salzartigen Rest darstellt,
umsetzt,
und/oder daß man gegebenenfalls eine Verbindung der Formel I nach Anspruch 1 aus einem ihrer funktionellen Derivate durch Behandeln mit einem solvolysierenden oder hydrogenolysierenden Mittel in Freiheit setzt, oder eine Verbindung der Formel I durch Reduktion oder Oxidation in eine andere Verbindung der Formel I umwandelt,
und/oder daß man eine Base der Formel I gemäß Anspruch 1 durch Behandeln mit einer Säure in eines ihrer Salze umwandelt.

5. Verfahren zur Herstellung pharmazeutischer Zubereitungen, dadurch gekennzeichnet, daß man eine Verbindung der Formel I gemäß Anspruch 1 und/oder eines ihrer physiologisch unbedenklichen Salze zusammen mit mindestens einem festen, flüssigen oder halbflüssigen Träger- oder Hilfsstoff in eine geeignete Dosierungsform bringt.

6. Pharmazeutische Zubereitung, gekennzeichnet durch einen Gehalt an mindestens einer Verbindung der Formel I gemäß Anspruch 1 und/oder einem ihrer physiologisch unbedenklichen Salze.

7. Verbindungen der Formel I gemäß Anspruch 1 und/oder ihre physiologischen unbedenklichen Salze zur Bekämpfung von Krankheiten.

8. Verwendung von Verbindungen der Formel I gemäß Anspruch 1 und/oder ihren physiologisch unbedenklichen Salzen bei der Bekämpfung von Krankheiten.

9. Verwendung von Verbindungen der Formel I gemäß Anspruch 1 und/oder ihren physiologisch unbedenklichen Salzen zur Herstellung von Arzneimitteln.

10. Verwendung von Verbindungen der Formel I gemäß Anspruch 1 und/oder ihren physiologisch unbedenklichen Salzen zur Herstellung von Neuroleptika.

## Claims

1. 4-Aryloxy- or 4-arylthiopiperidine derivatives of the formula I 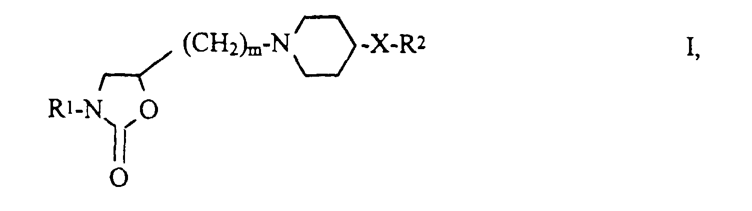 in which
R¹ and R² are each, independently of one another, phenyl radicals which are unsubstituted or mono- or disubstituted by A, OH, OA, aryloxy with 6-10 C atoms, aralkyloxy with 7-11 C atoms, -O- (CH₂)ₙ-O-, Hal, CF₃, NO₂, NH₂, NHA, NA₂, NHAc, NAAc, NHSO₂A and/or NASO₂A,
X is O, S, SO or SO₂,
m is 1, 2 or 3,
n is 1 or 2,
A is an alkyl radical with 1-6 C atoms,
Hal is F, Cl, Br or I
and
Ac is alkanoyl with 1-8 C atoms, aralkanoyl with 1-10 C atoms or aroyl with 7-11 C atoms,
and the physiologically acceptable salts thereof.

2. An enantiomer of a compound of the formula I accordinq to Claim 1.

3. (a) 3-p-chlorophenyl-5-[(4-p-acetamidophenoxy-piperidino)methyl]-2-oxazolidinone;
(b) 3-p-hydroxyphenyl-5-[(4-p-acetamidophenoxy-piperidino)methyl]-2-oxazolidinone;
(c) 3-p-methoxyphenyl-5-[(4-p-chlorophenoxy-piperidino)methyl]-2-oxazolidinone;
(d) 3-p-methoxyphenyl-5-[(4-p-hydroxyphenoxy-piperidino)methyl]-2-oxazolidinone;
(e) 3-p-methoxyphenyl-5-[(4-p-nitrophenoxy-piperidino)methyl]-2-oxazolidinone;
(f) 3-p-methoxyphenyl-5-[(4-p-chlorophenylthio-piperidino)methyl]-2-oxazolidinone;
(g) 3-p-methoxyphenyl-5-[(4-p-methylphenylthio-piperidino)methyl]-2-oxazolidinone;
(h) 3-p-methoxyphenyl-5-[(4-p-methoxyphenylsulfonylpiperidino)methyl]-2-oxazolidinone.
according to Claim 1.

4. Process for the preparation of compounds of the formula I according to Claim 1 and of the salts thereof, characterized in that a compound of the formula II 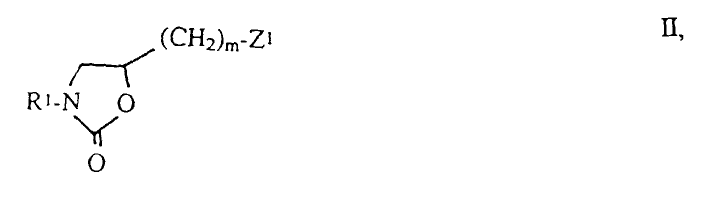 in which
R¹ and m have the meanings stated in Claim 1 and
Z¹ is Z or NH₂,
Z is Cl, Br, I, OH, SO₃CH₃ or another reactive functionally modified OH group,
if Z¹ = Z,
is reacted with a compound of the formula III 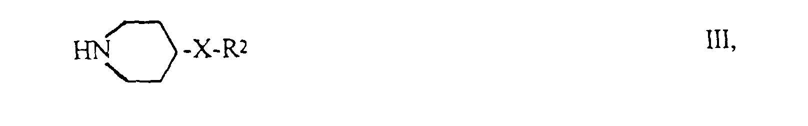 in which
R² and X have the meanings stated in Claim 1,
or, if Z¹ = NH₂,
with a compound of the formula IIIa 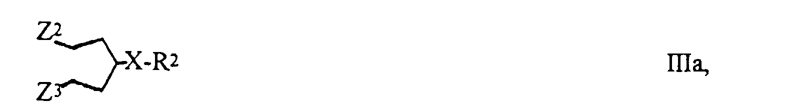 in which
R² and X have the meanings stated in Claim 1 and
Z² and Z³ are identical or different and each is Cl, Br, I, OH, SO₃CH₃ or another reactive functionally modified OH group,
or in that a compound which otherwise corresponds to the formula I but, in place of one or more hydrogen atoms, contains one or more reducible groups and/or one or more additional -SO₂- and/or -SO- groups is treated with a reducing agent,
or in that to prepare a compound of the formula I according to Claim 1 a radical R¹ and/or R² is converted into another radical R¹ and/or R²,
or in that a compound of the formula IV 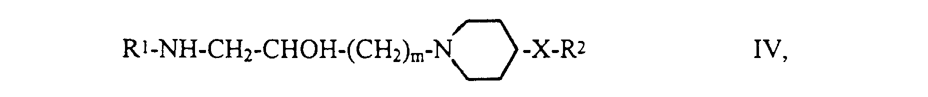 in which R¹, R², X and m have the stated meanings, is reacted with a suitable reactive carbonic acid derivative,
or in that a compound of the formula V 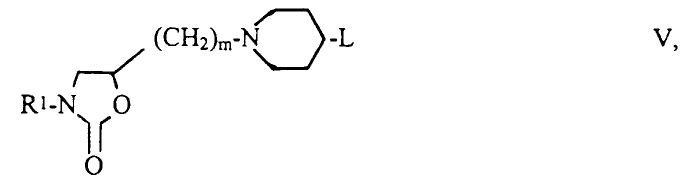 in which
R¹ and m have the stated meanings and
L is Cl, Br or another suitable leaving group,
is reacted with a compound of the formula VI
X¹-R² VI,
in which
R² has the stated meaning and
X¹ is OH, SO₂H, SH or a suitable salt-like radical derivable therefrom,
and/or in that, where appropriate, a compound of the formula I is liberated from one of its functional derivatives by treatment with a solvolysing or hydrolysing agent, or a compound of the formula I is converted by reduction or oxidation into another compound of the formula I,
and/or in that a base of the formula I according to Claim 1 is converted by treatment with an acid into one of its salts.

5. Process for the production of pharmaceutical preparations, characterized in that a compound of the formula I according to Claim 1 and/or one of its physiologically acceptable salts is converted together with at least one solid, liquid or semiliquid vehicle or ancillary substance into a suitable dosage form.

6. Pharmaceutical preparation characterized by a content of at least one compound of the formula I according to Claim 1 and/or one of its physiologically acceptable salts.

7. Compounds of the formula I according to Claim 1 and/or their physiologically acceptable salts for controlling diseases.

8. Use of compounds of the formula I according to Claim 1 and/or their physiologically acceptable salts for controlling diseases.

9. Use of compounds of the formula I according to Claim 1 and/or their physiologically acceptable salts for the production of medicaments.

10. Use of compounds of the formula I according to Claim 1 and/or their physiologically acceptable salts for the production of neuroleptics.

## Revendications

1. Dérivés de 4-aryloxy- ou de 4-arylthiopipéridines de formule I 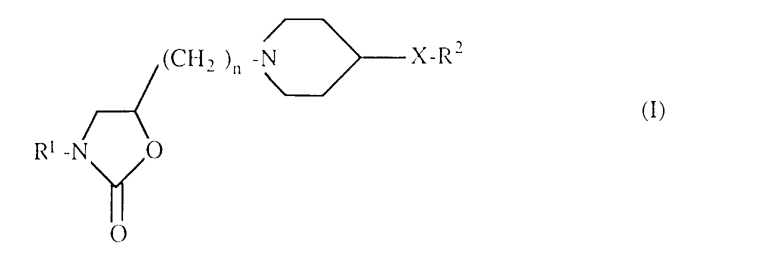 dans laquelle
R¹ et R² représentent chacun, indépendamment l'un de l'autre, un groupe phényle non substitué ou portant un ou deux substituants A, OH, OA, aryloxy en C₆₋₁₀, aralkyloxy en C₇₋₁₁, -O-(CH₂)ₙ-O-, Hal, CF₃, NO₂, NH₂, NHA, NA₂, NHAc, NHSO₂A, et/ou NASO₂A,
X représente O, S, SO ou SO₂,
m est égal à 1, 2 ou 3,
n est égal à 1 ou 2,
A représente un groupe alkylc en C₁₋₆,
Hal représente F, CL, Br ou I et
Ac représente un groupe alcanoyle en C₁₋₈, aralcanoyle en C₁₋₁₀ ou aroyle en C₇₋₁₁,
et leurs sels acceptables pour l'usage pharmaceutique.

2. Un énantiomère d'un composé de formule I selon la revendication 1.

3. (a) La 3-p-chlorophényl-5-[4-p-acétamidophénoxypipéridino)-méthyl]-oxa-zolidine-2-one ;
(b) la 3-p-hydroxyphényl-5-[(4-p-acétamidophénoxypipéridino)-méthyl]-oxazolidine-2-one ;
(c) la 3-p-méthoxyphényl-5-[(4-chlorophénoxypipéridino)-méthyl]-oxazolidine-2-one ;
(d) la 3-p-méthoxyphényl-5-[('-p-hydroxyphénoxypipéridino)-méthyl]-oxazolidine-2-one ;
(e) la 3-p-méthoxyphényl-5-[(4-p-nitrophénoxypipéridino)-méthyl]-oxazolidine-2-one ;
(f) la 3-p-méthoxyphényl-5-[(4-p-chlorophénylthiopipéridino)-méthyl]-oxazolidine-2-one ;
(g) la 3-p-méthoxyphényl-5-[(4-p-méthylphénylthiopipéridino)-méthyl]-oxazolidine-2-one ;
(h) la 3-p-méthoxyphényl-5-[(4-p-méthoxyphénylsulfonylpipéridino)-méthyl]-oxazolidine-2-one
selon la revendication 1.

4. Procédé de préparation des composés de formule I selon la revendication 1 et de leurs sels, caractérisé en ce que l'on fait réagir un composé de formule II 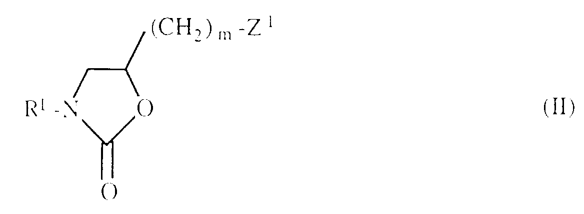 dans laquelle
R¹ et m ont les significations indiquées dans la revendication 1 et
Z¹ représente Z ou NH₂,
Z représente Cl, Br, I, OH, SO₃CH₃ ou un autre groupe OH ayant subi une modification fonctionnelle et réactif,
avec un composé de formule III 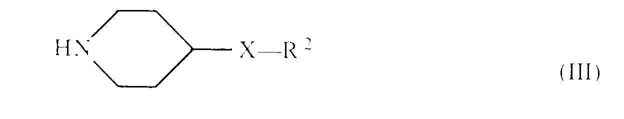 dans laquelle
R² et X ont les significations indiquées dans la revendication 1, lorsque Z¹ = Z,
avec un composé de formule IIIa, 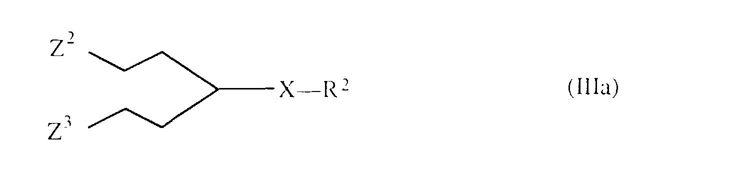 dans laquelle
R² et X ont les significations indiquées dans la revendication 1 et
Z² et Z³, ayant des significations identiques ou différentes, représentent chacun Cl, Br, I, OH, SO₃CH₃ ou un autre groupe OH ayant subi une modification fonctionnelle et réactif,
lorsque Z¹ = NH²,
ou bien on traite par un agent réducteur un composé répondant à la formule I mais dans laquelle, à la place d'un ou plusieurs atomes d'hydrogène, il y a un ou plusieurs groupes réductibles et/ou un ou plusieurs groupes -SO₂- et/ou -SO- supplémentaires,
ou bien encore, pour la préparation d'un composé de formule I selon la revendication 1, on convertit un substituant R¹ et/ou R² en un autre substituant R¹ et/ou R²,
ou bien on fait réagir un composé de formule IV 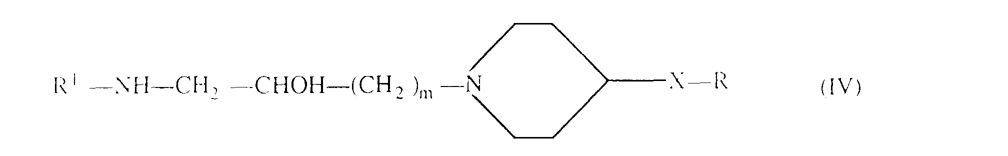 dans laquelle
R¹, R², X et m ont les significations indiquées ci-dessus, avec un dérivé réactif approprié de l'acide carbonique, ou bien on fait réagir un composé de formule V : 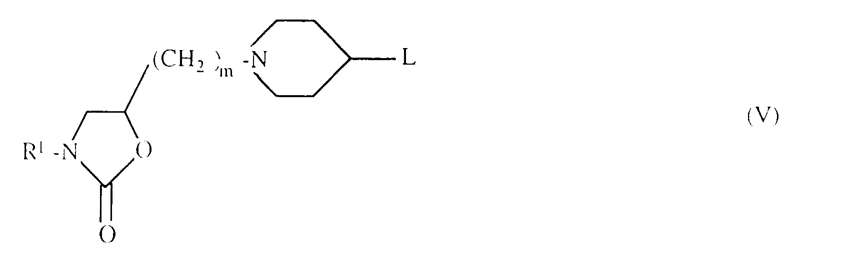 dans laquelle R¹ et m ont les significations indiquées ci-dessus et
L représente Cl, Br ou un autre substituant éliminable approprié,
avec un composé de formule VI
X¹-R²
dans laquelle
R¹ a les significations indiquées ci-dessus et
X représente OH, SO₂H, SH ou un groupe salin approprié, dérivant des groupes précédents, et/ou le cas échéant, on libère un composé de formule I selon la revendication 1 à partir de l'un de ses dérivés fonctionnels en le traitant par un agent solvolysant ou hydrogénolysant, ou bien on convertit un composé de formule I en un autre composé de formule I par réduction ou oxydation, ou bien on convertit une base de formule I selon la revendication 1 en l'un de ses sels en la traitant par un acide.

5. Procédé de préparation de compositions pharmaceutiques, caractérisé en ce que l'on met un composé de formule I selon la revendication 1 et/ou l'un de ses sels acceptables pour l'usage pharmaceutique sous une forme de dosage approprié avec au moins un véhicule ou produit auxiliaire solide, liquide ou semi-liquide.

6. Composition pharmaceutique caractérisée en ce qu'elle contient au moins un composé de formule I selon la revendication 1 et/ou l'un de ses sels acceptables pour l'usage pharmaceutique.

7. Composés de formule I selon la revendication 1 et/ou leurs sels acceptables pour l'usage pharmaceutique, pour le traitement de maladies.

8. Utilisation des composés de formule I selon la revendication 1 et/ou de leurs sels acceptables pour l'usage pharmaceutique pour le traitement de maladies.

9. Utilisation de composés de formule I selon la revendication 1 et/ou de leurs sels acceptables pour l'usage pharmaceutique pour la préparation de médicaments.

10. Utilisation de composés de formule I selon la revendication 1 et/ou de leurs sels acceptables pour l'usage pharmaceutique pour la préparation d'agents neuroleptiques.
